(19) Europäisches Patentamt
European Patent Office
Office européen des brevets

(11) EP 1 323 411 A1

(12) DEMANDE DE BREVET EUROPEEN

(43) Date de publication:
**02.07.2003 Bulletin 2003/27**

(51) Int Cl.[7]: **A61K 7/42**

(21) Numéro de dépôt: **02292837.8**

(22) Date de dépôt: **14.11.2002**

(84) Etats contractants désignés:
**AT BE BG CH CY CZ DE DK EE ES FI FR GB GR IE IT LI LU MC NL PT SE SK TR**
Etats d'extension désignés:
**AL LT LV MK RO SI**

(30) Priorité: **07.12.2001 FR 0115857**

(71) Demandeur: **L'OREAL**
**75008 Paris (FR)**

(72) Inventeur: **Candau, Didier**
**91570 Bievres (FR)**

(74) Mandataire: **Miszputen, Laurent**
**L'OREAL-DPI**
**6 rue Bertrand Sincholle**
**92585 Clichy Cédex (FR)**

# (54) Compositions cosmétiques antisolaires à base d'un mélange synergique de filtres et utilisations

(57) L'invention concerne de nouvelles compositions cosmétiques ou dermatologiques à usage topique, en particulier pour la photoprotection de la peau et/ou des cheveux, caractérisées par le fait qu'elles comprennent, dans un support cosmétiquement acceptable :

(i) au moins un dérivé silicié à fonction benzotriazole à titre de premier filtre ;
(ii) au moins un dérivé de dibenzoylméthane à titre de deuxième filtre ;
(iii) au moins au moins un dérivé de 2-hydroxybenzophénone amino-substitué de formule (III), à titre de troisième filtre. L'association de ces trois filtres conduit à l'obtention d'un effet de synergie au niveau des facteurs de protection UV-$A_{PPD}$ conférés.

L'invention concerne également leurs applications à la protection de la peau et des cheveux contre les effets du rayonnement ultraviolet.

EP 1 323 411 A1

## Description

L'invention concerne de nouvelles compositions cosmétiques ou dermatologiques à usage topique, en particulier pour la photoprotection de la peau et/ou des cheveux, caractérisées par le fait qu'elles comprennent, dans un support cosmétiquement acceptable, au moins :

(i) au moins un dérivé silicié à fonction benzotriazole à titre de premier filtre ;
(ii) au moins un dérivé de dibenzoylméthane à titre de deuxième filtre ;
(iii) au moins un dérivé de 2-hydroxybenzophénone amino-substitué particulier à titre de troisième filtre. L'association de ces trois filtres conduit à l'obtention d'un effet de synergie au niveau des facteurs de protection UV-$A_{PPD}$ conférés.

L'invention concerne également leurs applications à la protection de la peau et des cheveux contre les effets du rayonnement ultraviolet.
On sait que les radiations lumineuses de longueurs d'onde comprises entre 280 nm et 400 nm permettent le brunissement de l'épiderme humain et que les rayons de longueurs d'onde comprises entre 280 et 320 nm, connus sous la dénomination d'UV-B, provoquent des érythèmes et des brûlures cutanées qui peuvent nuire au développement du bronzage naturel ; ce rayonnement UV-B doit donc être filtré.
On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement prématuré. Ils favorisent le déclenchement de la réaction érythémateuse ou amplifient cette réaction chez certains sujets et peuvent même être à l'origine de réactions photo toxiques ou photo allergiques. Il est donc souhaitable de filtrer aussi le rayonnement UV-A.
De nombreuses compositions cosmétiques destinées à la photoprotection (UV-A et/ou UV-B) de la peau ont été proposées à ce jour.
L'efficacité des compositions anti-solaires s'exprime généralement par le facteur de protection solaire (FPS) s'exprimant mathématiquement par le rapport de la dose de rayonnement UV nécessaire pour atteindre le seuil érythématogène avec le filtre UV avec la dose de rayonnement UV nécessaire pour atteindre le seuil érythématogène sans filtre UV. Ce facteur concerne donc l'efficacité de la protection vis à vis de l'érythème dont le spectre d'action biologique est centré dans l'UVB et par conséquent, rend compte de la protection vis à vis de ce rayonnement UV-B.
Compte tenu des effets des UV-A sur la peau et du développement de nombreuses compositions contenant des associations de filtres capables d'absorber le rayonnement UV-B et/ou UV-A; il s'est développé des méthodes spécifiques d'évaluation de la protection contre le rayonnement UV-A.
Pour caractériser la protection vis à vis des UV-A, la méthode PPD (Persistent Pigment Darkening), qui mesure la couleur de la peau observée 2 à 4 heures après une exposition de la peau aux UV-A, est particulièrement recommandée et utilisée. Cette méthode est adoptée depuis 1996 par la Japanese Cosmetic Industry Association (JCIA) en tant que procédure officielle de test pour l'étiquetage UV-A des produits et est fréquemment utilisée par les laboratoires de tests en Europe et aux Etats-Unis; (Japan Cosmetic Industry Association Technical Bulletin. Measurement Standards for UVA protection efficacy. Issued November 21, 1995 and efective of January 1, 1996).
Le facteur de protection solaire $UVA_{PPD}$ (FP $UVA_{PPD}$) s'exprime mathématiquement par le rapport de la dose de rayonnement UV-A nécessaire pour atteindre le seuil de pigmentation avec le filtre UV ($MPPD_p$) avec la dose de rayonnement UV-A nécessaire pour atteindre le seuil de pigmentation sans filtre UV ($MPPD_{np}$).

$$\text{FP UVA}_{PPD} = \frac{MPPD_p}{MPPD_{np}}$$

Ces compositions antisolaires se présentent assez souvent sous la forme d'une émulsion de type huile-dans-eau (c'est à dire un support cosmétiquement acceptable constitué d'une phase continue dispersante aqueuse et d'une phase discontinue dispersée huileuse) qui contient, à des concentrations diverses, un ou plusieurs filtres organiques classiques, lipophiles et/ou hydrophiles, capables d'absorber sélectivement les rayonnements UV nocifs, ces filtres (et leurs quantités) étant sélectionnés en fonction des facteurs de protection solaire recherchés.
On connaît dans l'état de la technique des filtres UV cosmétiques des dérivés siliconés à fonction benzotriazole lipophiles présentant de bonnes propriétés filtrantes aussi bien dans le domaine des radiations UV-A que dans le domaine des radiations UV-B. Ils sont décrits dans les demandes EP-A-0392883 ; EP-A-0660701 ; EP-A-0708108 ; EP-A-0711778 ; EP-A-711779.
Dans les demandes EP-A-0742003 et EP-A-0860165, on a déjà proposé d'associer à ces filtres silicones à

fonction benzotriazole des filtres hydrosolubles à fonction sulfonique particuliers à savoir l'acide benzène 1,4-di(3-méthylidène-10-camphosulfonique) ou l'acide 2-phényl benzimidazole 5-sulfonique et ses sels, en vue de produire une activité synergique au niveau des indices de protection solaire. Ces systèmes filtrants synergiques imposent l'utilisation d'au moins une phase aqueuse solubilisant le filtre hydrosoluble et d'une phase grasse pour solubiliser le filtre siliconé ; ce qui réduit sensiblement les possibilités de formulation.

On connaît dans les demandes de brevet EP-A-1046391 et DE10012408 des compositions solaires à base de dérivés de 2-hydroxy benzophénone aminosubstitués pouvant contenir d'autres filtres complémentaires tels que des dérivés siliconés à fonction benzotriazole ou des dérivés de dibenzoylméthane.

A la suite d'importantes recherches menées dans le domaine de la photoprotection évoqué ci-dessus, la Demanderesse a découvert, de façon inattendue et surprenante, qu'une combinaison de trois familles de composés filtrants particulières et déjà connues en soi dans l'état de l'art (1) un dérivé de dibenzoylméthane, (2) un dérivé silicié de benzotriazole et (3) un dérivé de 2-hydroxy benzophénone aminosubstitué particulier permettait, du fait d'un effet de synergie, d'obtenir des compositions antisolaires présentant des facteurs de protection solaire $UVA_{PPD}$ nettement améliorés. Une telle association permettait d'obtenir des compositions solaires dont les facteurs de protection solaire $UVA_{PPD}$ sont nettement améliorés, et en tous cas largement supérieurs à ceux qui peuvent être obtenus soit avec l'un ou l'autre des filtres utilisé seul.

De plus, l'association particulière de filtres conforme à l'invention peut être facilement incorporée dans une très large gamme de supports cosmétiques.

Cette découverte est à la base de la présente invention.

Ainsi, conformément à l'un des objets de la présente invention, il est maintenant proposé de nouvelles compositions cosmétiques, en particulier antisolaires, qui sont caractérisées par le fait qu'elles comprennent, dans un support cosmétiquement acceptable :

(i) au moins un dérivé silicié à fonction benzotriazole à titre de premier filtre ;
(ii) au moins un dérivé de dibenzoylméthane à titre de deuxième filtre ;
(iii) au moins un dérivé de 2-hydroxy benzophénone aminosubstitué de formule (III) telle que définie ci-dessous, à titre de troisième filtre.

La présente invention a également pour objet l'utilisation des compositions ci-dessus pour la fabrication de compositions cosmétiques destinées à la protection de la peau et/ou des cheveux contre le rayonnement ultraviolet, en particulier le rayonnement solaire.

De façon générale lesdits premier, second et troisième filtres sont présents dans les compositions de l'invention dans une proportion produisant une activité synergique au niveau des facteurs de protection solaire $UVA_{PPD}$ conférés .

D'autres caractéristiques, aspects et avantages de la présente invention apparaîtront à la lecture de la description détaillée qui va suivre.

Les dérivés siliciés à fonction benzotriazole utilisés dans la présente invention sont de préférence des silanes ou des siloxanes à fonction benzotriazole comprenant au moins une unité de formule (1) suivante :

$$O_{(3-a)/2}Si\ (R_7)_a\ -\ G \qquad (1)$$

dans laquelle :

- $R_7$ représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante :

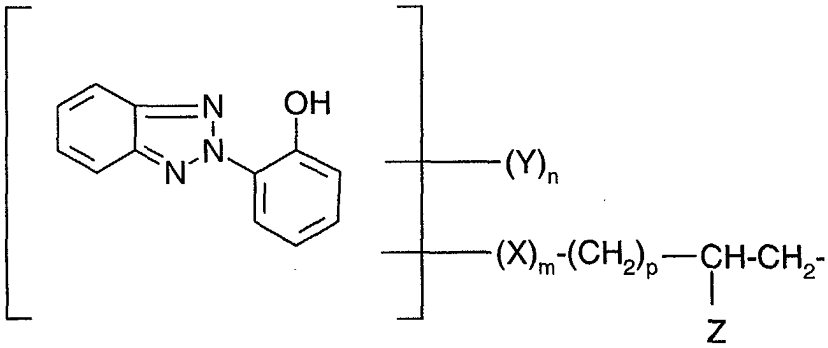

(2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

Ces composés sont notamment décrits dans les demandes de brevet EP-A-0392883 ; EP-A-0660701 ; EP-A-0708108 ; EP-A-0711778 ; EP-A-711779.

De préférence, les dérivés siliciés utilisés dans le cadre de la présente invention appartiennent à la famille générale des silicones benzotriazoles qui est décrite notamment dans EP-A-0660701 .

Une famille de silicones benzotriazoles convenant particulièrement bien à la réalisation de la présente invention est celle regroupant les composés répondant aux formules (5) ou (6) suivantes :

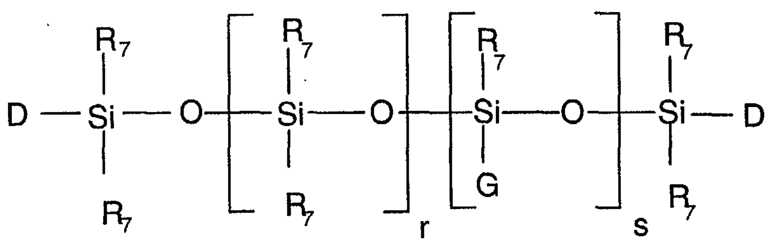

(5)

ou

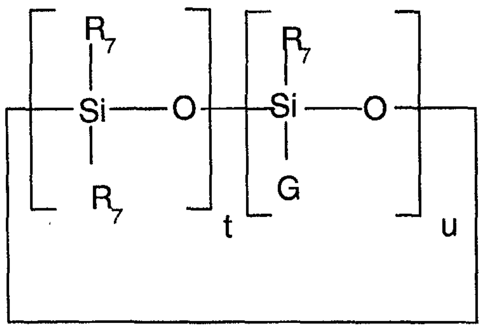

(6)

dans lesquelles :

- $R_7$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_7$ étant méthyle,
- D, identiques ou différents sont choisis parmi les radicaux $R_7$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) ci-dessus.

Comme cela ressort de la formule (2) donnée ci-dessus, l'accrochage du chaînon $-(X)_m-(CH_2)_p-CH(Z)-CH_2-$ sur le motif benzotriazole, qui assure donc le raccordement dudit motif benzotriazole à l'atome de silicium de la chaîne siliconée, peut, selon la présente invention, se faire dans toutes les positions disponibles offertes par les deux noyaux aromatiques du benzotriazole :

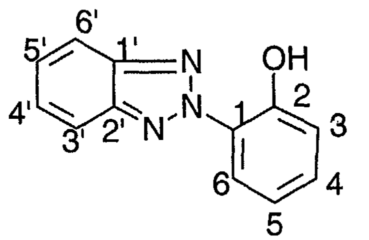

De préférence, cet accrochage se fait en position 3, 4, 5 (noyau aromatique portant la fonction hydroxy) ou 4' (noyau benzénique adjacent au cycle triazolé), et encore plus préférentiellement en position 3, 4 ou 5. Dans une forme préférée de réalisation de l'invention, l'accrochage se fait en position 3.

De même, l'accrochage du ou des motifs substituants Y peut se faire dans toutes les autres positions disponibles au sein du benzotriazole. Toutefois, de préférence, cet accrochage se fait en position 3, 4, 4', 5 et/ou 6. Dans une forme préférée de réalisation de l'invention, l'accrochage du motif Y se fait en position 5.

Dans les formules (5) et (6) ci-dessus, les radicaux alkyle peuvent être linéaires ou ramifiés et choisis notamment au sein des radicaux méthyle, éthyle, n-propyle, isopropyle, n-butyle, isobutyle, ter.-butyle, n-amyle, isoamyle, néopentyle, n-hexyle, n-heptyle, n-octyle, éthyl-2 hexyle et tert-octyle. Les radicaux alkyle $R_7$ préférés selon l'invention sont les radicaux méthyle, éthyle, propyle, n-butyle, n-octyle et éthyl-2 hexyle. Encore plus préférentiellement, les radicaux $R_7$ sont tous des radicaux méthyle.

Parmi les composés de formules (5) ou (6) ci-dessus, on préfère mettre en oeuvre ceux répondant à la formule (5), c'est-à-dire des diorganosiloxanes à chaîne courte linéaire.

Parmi les composés de formule (5) ci-dessus, on préfère mettre en oeuvre ceux pour lesquels les radicaux D sont tous les deux des radicaux $R_7$

Parmi les diorganosiloxanes linéaires rentrant dans le cadre de la présente invention, on préfère plus particulièrement les dérivés statistiques ou bien définis à blocs présentant au moins l'une, et encore plus préférentiellement

l'ensemble, des caractéristiques suivantes :

- D est un radical $R_7$
- $R_7$ est alkyle et encore plus préférentiellement est méthyle,
- r est compris entre 0 et 15 inclusivement ; s est compris entre 1 et 10 inclusivement,
- n est non nul, et de préférence égal à 1, et Y est alors choisi parmi méthyle, tert.-butyle ou alcoxy en $C_1$-$C_4$,
- Z est hydrogène ou méthyle,
- m=0, ou [m=1 et X=O]
- p est égal à 1.

Une famille de silicones benzotriazoles convenant particulièrement bien à l'invention est celle définie par la formule générale (7) suivante :

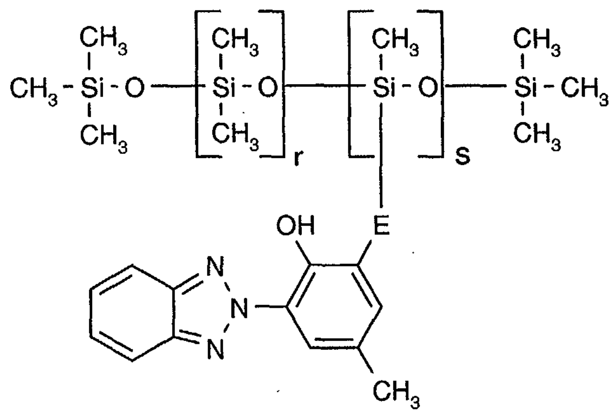

(7)

avec

$$0 \leq r \leq 10,$$

$$1 \leq s \leq 10,$$

et où E représente le radical divalent :

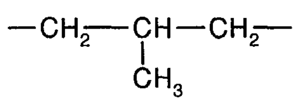

Dans une forme particulièrement préférée de réalisation de l'invention, la silicone benzotriazole est le composé Drométrizole Trisiloxane (nom CTFA) répondant à la formule suivante :

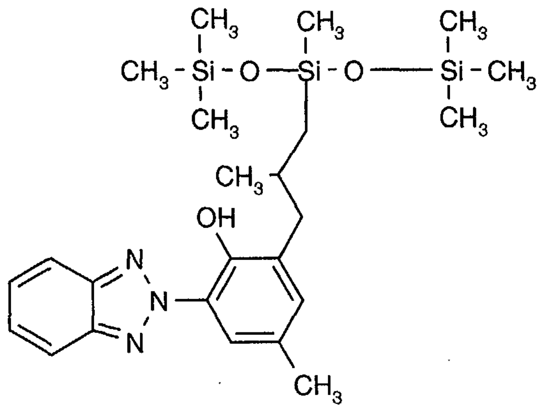

Des procédés convenant à la préparation des produits de formules (1), (5), (6) et (7) ci-dessus sont notamment décrits dans les brevets américains US3,220,972, US3,697,473, US4,340,709, US4,316,033, US4,328,346 et dans les demandes de brevet EP-A-0392883 et EP-A-0742 003.

Le dérivé silicié à fonction benzotriazole peut être présent dans les compositions selon l'invention à des teneurs allant de 0,5 à 20%, de préférence allant de 1 à 10%, en poids, et plus particulièrement de 2 à 8% en poids toujours par rapport au poids total de la composition.

Comme indiqué précédemment, les dérivés du dibenzoylméthane visés par la présente invention sont des produits déjà bien connus en soi et décrits notamment dans les documents FR-A- 2 326 405, FR-A- 2 440 933 et EP-A-0 114 607, documents dont les enseignements sont, pour ce qui touche à la définition même de ces produits, totalement inclus à titre de références dans la présente description.

Parmi les dérivés du dibenzoylméthane plus particulièrement visés par la présente invention, on peut notamment citer, de manière non limitative :

- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- 4-tert-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane.

Parmi les dérivés du dibenzoylméthane mentionnés ci-dessus, on préfère tout particulièrement, selon la présente invention, mettre en oeuvre le 4-tert-butyl-4'-méthoxydibenzoylméthane, notamment celui proposé à la vente sous la dénomination commerciale de "PARSOL 1789" par la Société Hoffmann Laroche, ce filtre répondant à la formule développée (I) suivante :

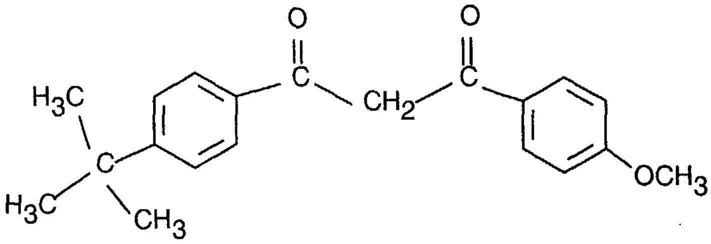

(I)

Un autre dérivé du dibenzoylméthane préféré selon la présente invention est le 4-isopropyldibenzoylméthane, filtre vendu sous la dénomination de "EUSOLEX 8020" par la Société Merck, et répondant à la formule développée (II) suivante :

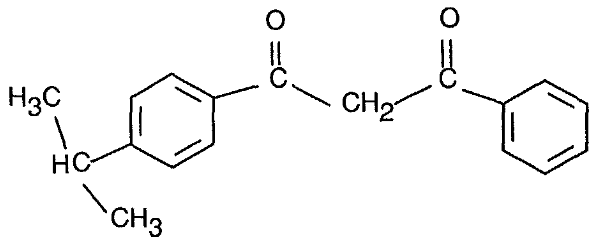

(II)

Le ou les dérivés du dibenzoylméthane sont présents dans les compositions conformes à l'invention à des teneurs de préférence allant de 0,5 à 20% en poids et plus préférentiellement de 1 % à 10 % en poids, et plus particulièrement de 2 à 8% en poids par rapport au poids total de la composition.

Les dérivés de 2-hydroxybenzophénone aminosubstitués conformes à l'invention répondent à la formule (III) suivante :

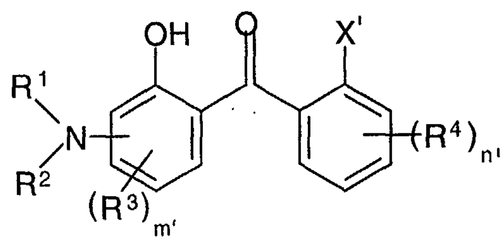

(III)

dans laquelle :

$R^1$ et $R^2$, identiques ou différents, désignent un atome d'hydrogène, un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$ ;
$R^1$ et $R^2$ peuvent également former avec l'atome d'azote avec lequel ils sont liés un cycle à 5 ou 6 chaînons ;
$R^3$ et $R^4$, identiques ou différents, désignent un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$, un radical alcoxy en $C_1$-$C_{12}$, un radical ($C_1$-$C_{20}$) alcoxy-carbonyle, un radical alkylamino en $C_1$-$C_{12}$, un radical dialkylamino en $C_1$-$C_{12}$, un radical aryle ou un hétéroaryle éventuellement substitué, un substituant hydrosolubilisant choisi parmi un groupe carboxylate, un groupe sulfonate ou un reste ammonium ;
X' désigne un atome d'hydrogène, un groupe $COOR^5$ ou $CONR^6R^7$ ;
$R^5$, $R^6$ et $R^7$, identiques ou différents, désignent un atome d'hydrogène, un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$, un groupe $-(Y'O)_o$-Z' ou un groupe aryle ;
Y' désigne $-(CH_2)_2-$, $-(CH_2)_3-$ $-(CH_2)_4-$, $-CH-(CH_3)-CH_2-$ ;
Z' représente $-CH_2-CH_3$, $-CH_2CH_2CH_3-$, $-CH_2-CH_2-CH_2-CH_3$, $-CH(CH_3)-CH_3$ ;
m' est un entier variant de 0 à3 ;
n' est un entier variant de 0 à 3 ;
o est un entier variant de 1 à 2.

Comme radicaux alkyle en $C_1$-$C_{20}$, on peut citer par exemple : méthyle, éthyle, n-propyle, 1-méthyléthyle, n-butyle, 1-méthylpropyle, 2-méthylpropyle, 1,1-diméthyléthyle, n-pentyle, 1-méthylbutyle, 2-méthylbutyle, 3-méthylbutyle, 2,2-diméthylpropyle, 1-éthylpropyle, n-hexyle, 1,1-diméthylpropyle, 1,2-diméthylpropyle, 1-méthylpentyle, 2-méthylpentyle, 3-méthylpentyle, 4-méthylpentyle, 1,1-diméthylbutyle, 1,2-diméthylbutyle, 1,3-diméthylbutyle, 2,2-diméthylbutyle, 2,3-diméthylbutyle, 3,3-diméthylbutyle, 1-éthylbutyle, 2-éthylbutyle, 1,2,2-triméthylpropyle, 1-éthyl-1-méthylpropyle, 1-éthyl-2-méthylpropyle, n-heptyle, n-octyle, n-nonyle, n-décyle, n-undécyle, n-dodécyle, n-tridécyle, n-tétradécyle, n-pentadécyle, n-hexadécyle, n-heptadécyle, n-octadécyle, n-nonadécyle ou n-eicosyle.

Comme groupes alcènyle en $C_2$-$C_{10}$, on peut citer par exemple : vinyle, n-propènyle, isopropènyle, 1-butènyle, 2-butènyle, 1-pentènyle, 2-pentènyle, 2-méthyl-1-butènyle, 2-méthyl-2-butènyle, 3-méthyl-1-butènyle, 1-hexènyle, 2-hexènyle, 1-heptènyle, 2-heptènyle, 1-octènyle, 2-octènyle.

Comme radicaux alcoxy en $C_1$-$C_{12}$, on peut citer : méthoxy, éthoxy, n-propoxy, n-butoxy, n-pentoxy, 1-méthylpropoxy, 3-méthylbutoxy, 2,2-diméthylpropoxy, 1-méthyl- 1-éthylpropoxy, octoxy, 2-méthylpropoxy, 1,1-diméthylpropoxy, hexoxy, heptoxy, 2-éthylhexoxy.

Comme radicaux cycloalkyles en $C_3$-$C_{10}$, on peut citer par exemple : cyclopropyle, cyclobutyle, cyclopentyle, cyclohexyle, cycloheptyle, 1-méthylcyclopropyle, 1-éthylcyclopropyle, 1-propylcyclopropyle, 1-butylcyclopropyle, 1-pentylcyclopropyle, 1-méthyl-1-butylcyclopropyle, 1,2-diméthylcyclopropyle, 1-méthyl-2-éthylcyclopropyle, cyclooctyle, cyclononyle ou cyclodécyle.

Comme radicaux cycloalcènyles en $C_3$-$C_{10}$ ayant une ou plusieurs doubles liaisons, on peut citer : cyclopropènyle, cyclobutènyle, cyclopentènyle, cyclopentadiènyle, cyclohexènyle, 1,3-cyclohexadiènyle, 1,4-cyclohexadiènyle, cycloheptènyle, cycloheptatriènyle, cyclooctènyle, 1,5-cyclooctadiènyle, cyclooctatétraènyle, cyclononènyle ou cyclodécènyle.

Les radicaux cycloalkyles ou cycloalcényles peuvent comporter un ou plusieurs substituants (de préférence de 1 à 3) choisis par exemple parmi les halogène comme chlore, fluor ou brome; cyano; nitro; amino; $C_1$-$C_4$-alkylamino; $C_1$-$C_4$ dialkylamino; $C_1$-$C_4$alkyle; $C_1$-$C_4$-alcoxy; hydroxy; ils peuvent également comporter de 1 à 3 hétéroatomes comme souffre, oxygène ou azote dont les valences libres peuvent être occupées par un hydrogène ou un radical alkyle en $C_1$-$C_4$.

Les groupes aryles sont de préférence choisis parmi les cycles phényle ou naphtyle, lesquels peuvent comporter un ou plusieurs substituants (de préférence de 1 à 3) choisis par exemple parmi les halogènes comme chlore, fluor ou brome ; cyano ; nitro ; amino ; $C_1$-$C_4$-alkylamino ; $C_1$-$C_4$ dialkylamino ; $C_1$-$C_4$alkyle ; $C_1$-$C_4$-alcoxy ; hydroxy. On préfère plus particulièrement phényle, méthoxyphényle et naphtyle.

Les groupes hétéroaryles comportent en général un ou plusieurs hétéroatomes choisis parmi souffre, oxygène ou azote.

Les groupes hydrosolubilisants sont par exemple des groupes carboxylates, sulfonates et plus particulièrement leurs sels avec des cations physiologiquement acceptables comme les sels de métaux alcalins ou les sels de trialkylammonium comme les sels de tri(hydroxyalkyl)ammonium ou de 2-méthylpropan-1-ol-2-ammonium. On peut également citer les groupes ammonium comme les alkylammoniums et leurs formes salifiées avec des anions physiologiquement acceptables.

Comme exemples de cycle à 5 ou 6 chaînons formé par les radicaux $R^1$ et $R^2$ avec l'atome d'azote, on peut citer en particulier pyrrolidine ou pipéridine.

Les groupes amino peuvent être fixés sur le noyau benzénique en position ortho, méta ou para par rapport au radical carbonyle et plus préférentiellement en para.

**[0052]** Une famille de composés de formule (III) préférentiels comprend ceux choisis parmi ceux de formule (IIIa) suivante :

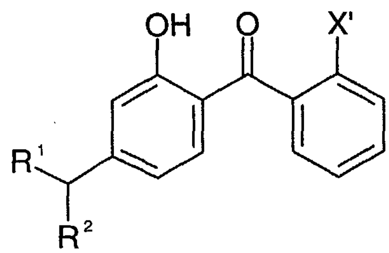

(IIIa)

dans laquelle :

$R^1$ et $R^2$, identiques ou différents, désignent un atome d'hydrogène, un radical alkyle en $C_1$-$C_{12}$ ou forment avec l'atome d'azote avec lequel ils sont liés un cycle à 5 ou 6 chaînons ;
X' désigne $COOR^5$ ou $CONR^6R^7$;
$R^5$ désigne un atome d'hydrogène, un radical alkyle en $C_1$-$C_{12}$, un radical cycloalkyle en $C_3$-$C_6$.
$R^6$ et $R^7$, identiques ou différents, désignent un atome d'hydrogène, un radical alkyle en $C_1$-$C_{12}$, un radical cycloalkyle en $C_5$-$C_6$.

Les composés de formule (IIIa) plus particulièrement préférés sont ceux pour lesquels :

$R^1$ et $R^2$, identiques ou différents, désignent un radical alkyle en $C_1$-$C_4$ et plus particulèrement éthyle ;
$R^5$ désigne un radical alkyle en $C_3$-$C_8$,
$R^6$ et $R^7$, identiques ou différents, désignent un radical alkyle en $C_1$-$C_8$,

Une autre famille de composés de formule (III) préférentiels comprend ceux choisis parmi ceux de formule (IIIb) suivante :

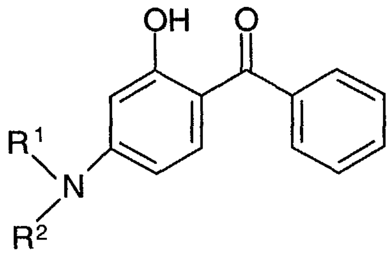

(IIIb)

dans laquelle :

$R^1$ et $R^2$, identiques ou différents, désignent un radical alkyle en $C_1$-$C_{12}$ ou forment avec l'atome d'azote avec lequel ils sont liés un cycle à 5 ou 6 chaînons.

Parmi les composés de formule (IIIb), on peut citer plus particulièrement :

- le (4-diéthylamino-2-hydroxyphényl)-phénylcétone.
- le (4-pyrrolidino-2-hydroxyphényl)-phénylcétone.

Une famille de composés de formule (III) plus particulièrement préférés comprend ceux choisis parmi ceux de formule (IIIc) suivante :

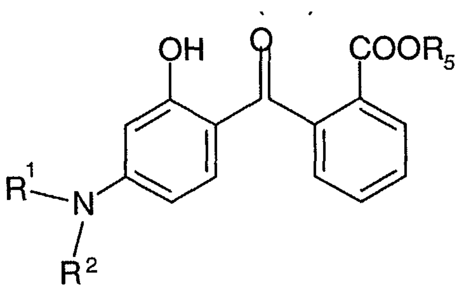

(IIIc)

dans laquelle :

$R^1$ et $R^2$, identiques ou différents, désignent un atome d'hydrogène, un radical alkyle en $C_1$-$C_8$ ou forment avec l'atome d'azote avec lequel ils sont liés un cycle à 5 ou 6 chaînons ;
$R^5$ désigne un atome d'hydrogène, un radical alkyle en $C_1$-$C_{12}$, un radical cycloalkyle en $C_3$-$C_6$.

Parmi les composés de formule (IIIc), on peut citer :

- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate

- le 2-(4-dibutylamino-2-hydroxybenzoyl)-benzoate de méthyle
- le 2-(4-dibutylamino-2-hydroxybenzoyl)-benzoate d'isobutyle.

Un composé de formule (III) tout particulièrement préféré est le 2-(4-diéthylamino-2-hydroxybenzoyl)-benzoate de n-hexyle.

Les composés de formule (III) tels que définis ci-dessus sont connus en eux-mêmes et leurs structures et leurs synthèses sont décrites dans les demandes de brevet EP1046391 et DE100 12 408 (faisant partie intégrante du contenu de la description).

Les dérivés de 2-hydroxybenzophénone aminosubstitués conformes à l'invention sont présents de préférence dans la composition de l'invention dans des proportions allant de 0,1 à 20 % en poids et plus préférentiellement de 0,1 à 15% en poids et plus particulièrement de 0,5 à 10% en poids par rapport au poids total de la composition.

Les compositions conformes à l'invention peuvent comporter en plus d'autres filtres UV organiques complémentaires actifs dans l'UVA et/ou l'UVB (absorbeurs), hydrosolubles ou liposolubles ou bien insolubles dans les solvants cosmétiques couramment utilisés.

Les filtres UV organiques complémentaires sont notamment choisis parmi les anthranilates ; les dérivés cinnamiques; les dérivés salicyliques, les dérivés du camphre ; les dérivés de triazine tels que ceux décrits dans les demandes de brevet US 4367390, EP863145, EP517104, EP570838, EP796851, EP775698, EP878469 et EP933376 ; les dérivés de la benzophénone autres que ceux de formule (III) définie ci-dessus ; les dérivés de β,β-diphénylacrylate, les dérivés de benzotriazole ; les dérivés de benzalmalonate ; les dérivés de benzimidazole ; les imidazolines; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE 197 26 184 et EP893119 ; les polymères filtres et silicones filtres autres que ceux de l'invention tels que ceux décrits notamment dans la demande WO-93/04665 ; les dérivés d'alkylstyrène tels que ceux décrits dans la demande de brevet DE19855649 ; les dérivés de 4,4-diarylbutadiène tels que ceux décrits dans les demandes de brevet EP0967200, DE19755649, EP1333981 ; et leurs mélanges.

Comme exemples de filtres organiques complémentaires actifs dans l'UV-A et/ou l'UV-B, on peut citer désignés ci-dessus sous leur nom INCI :

<u>Dérivés de l'acide para-aminobenzoique :</u>

- PABA,
- Ethyl PABA,
- Ethyl Dihydroxypropyl PABA,
- Ethylhexyl Diméthyl PABA vendu notamment sous le nom « ESCALOL 507 » par ISP,
- Glyceryl PABA,
- PEG-25 PABA vendu sous le nom « UVINUL P25 » par BASF,

<u>Dérivés salicyliques</u>:

- Homosalate vendu sous le nom « EUSOLEX HMS » par RONA/EM INDUSTRIES,
- Ethylhexyl Salicylate vendu sous le nom « NEO HELIOPAN OS » par HAARMANN et REIMER,
- Dipropyleneglycol Salicylate vendu sous le nom « DIPSAL » par SCHER,
- TEA Salicylate, vendu sous le nom « NEO HELIOPAN TS » par HAARMANN et REIMER,

<u>Dérivés cinnamigues:</u>

- Ethylhexyl Methoxycinnamate vendu notamment sous le nom commercial « PARSOL MCX » par HOFFMANN LA ROCHE,
- Isopropyl Methoxy cinnamate,
- Isoamyl Methoxy cinnamate vendu sous le nom commercial « NEO HELIOPAN E 1000 » par HAARMANN et REIMER,
- Cinoxate,

- DEA Methoxycinnamate,
- - Diisopropyl Methylcinnamate,
- Glyceryl Ethylhexanoate Dimethoxycinnamate

Dérivés de β,β'-diphénylacrylate :

- Octocrylene vendu notamment sous le nom commercial « UVINUL N539 » par BASF,
- Etocrylene, vendu notamment sous le nom commercial « UVINUL N35 » par BASF,

Dérivés de la benzophénone :

- Benzophenone-1 vendu sous le nom commercial « UVINUL 400 » par BASF,
- Benzophenone-2 vendu sous le nom commercial « UVINUL D50 » par BASF
- Benzophenone-3 ou Oxybenzone, vendu sous le nom commercial « UVINUL M40 » par BASF,
- Benzophenone-4 vendu sous le nom commercial « UVINUL MS40 » par BASF,
- Benzophenone-5
- Benzophenone-6 vendu sous le nom commercial « HELISORB 11 » par NORQUAY
- Benzophenone-8 vendu sous le nom commercial « SPECTRA-SORB UV-24 » PAR AMERICAN CYANAMID
- Benzophenone-9 vendu sous le nom commercial« UVINUL DS-49» par BASF,
- Benzophenone-12

Dérivés du benzylidène camphre :

- 3-Benzylidene camphor fabriqué sous le nom « MEXORYL SD» par CHIMEX,
- Benzylidene Camphor Sulfonic Acid fabriqué sous le nom « MEXORYL SL» par CHIMEX,
- Camphor Benzalkonium Methosulfate fabriqué sous le nom « MEXORYL SO » par CHIMEX,
- -Terephthalylidene Dicamphor Sulfonic Acid fabriqué sous le nom « MEXORYL SX » par CHIMEX,
- Polyacrylamidomethyl Benzylidene Camphor fabriqué sous le nom « MESORYL SW » par CHIMEX,

Dérivés de benzimidazole :

- Phenylbenzimidazole Sulfonic Acid vendu notamment sous le nom commercial « EUSOLEX 232 » par MERCK,
- Disodium Phenyl Dibenzimidazole Tetra-sulfonate vendu sous le nom commercial « NEO HELIOPAN AP » par HAARMANN et REIMER,

Dérivés de triazine :

- Anisotriazine vendu sous le nom commercial «TINOSORB S » par CIBA SPECIALTY CHEMICALS
- Ethylhexyl triazone vendu notamment sous le nom commercial «UVINUL T150 » par BASF,
- Diethylhexyl Butamido Triazone vendu sous le nom commercial « UVASORB HEB » par SIGMA 3V,
- la 2,4,6-tris-(4'-amino benzalmalonate de diisobutyle)-s- triazine.

Dérivés du phenyl benzotriazole :

- Methylène bis-Benzotriazolyl Tetramethylbutylphénol, vendu sous forme solide sous le nom commercial « MIXXIM BB/100 » par FAIRMOUNT CHEMICAL ou sous forme micronisé en dispersion aqueuse sous le nom commercial

« TINOSORB M » par CIBA SPECIALTY CHEMICALS,

Dérivés anthraniliques :

- Menthyl anthranilate vendu sous le nom commercial commercial « NEO HELIOPAN MA » par HAARMANN et REIMER,

Dérivés d'imidazolines :

- Ethylhexyl Dimethoxybenzylidene Dioxoimidazoline Propionate,

Dérivés de benzalmalonate :

- Polyorganosiloxane à fonction benzalmalonate vendu sous la dénomination commerciale « PARSOL SLX » par HOFFMANN LA ROCHE et leurs mélanges.

Dérivés de 4,4-diarylbutadiène :

- 1,1-dicarboxy (2,2'-diméthyl-propyl)-4,4-diphénylbutadiène

et leurs mélanges.

Les filtres UV organiques solubles plus particulièrement préférés sont choisis parmi les composés suivants :

- Ethylhexyl Salicylate,
- Ethylhexyl Methoxycinnamate,
- Octocrylene,
- Phenylbenzimidazole Sulfonic Acid,
- Benzophenone-3,
- Benzophenone-4,
- Benzophenone-5,
- 4-Methylbenzylidene camphor,
- Disodium Phenyl Dibenzimidazole Tetra-sulfonate,
- Anisotriazine,
- Ethylhexyl triazone,
- Diethylhexyl Butamido Triazone,
- la 2,4,6-tris-(4'-amino benzalmalonate de diisobutyle)-s- triazine.
- Methylène bis-Benzotriazolyl Tetramethylbutylphénol
- - 1,1-dicarboxy (2,2'-diméthyl-propyl)-4,4-diphénylbutadiène

et leurs mélanges.

Les compositions cosmétiques selon l'invention peuvent encore contenir des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP-A-0518772 et EP-A-0518773.

Les compositions selon l'invention peuvent également contenir des agents de bronzage et/ou de brunissage artificiel de la peau (agents autobronzants), tels que par exemple de la dihydroxyacétone (DHA).

Les compositions de l'invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones, les $\alpha$-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants ou tout autre ingrédient habituellement utilisé en cosmétique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C. Ils comprennent également les acides gras, les alcools gras et les esters d'acides gras, linéaires ou cycliques tels que les dérivés d'acide benzoïque, trimellitique et hydroxy-benzoïque.

Comme huiles, on peut citer les huiles minérales (paraffine); végétales (huile d'amande douce, de macadamia, de pépin de cassis, de jojoba) ; synthétiques comme le perhydrosqualène, les alcools, les acides ou les esters gras (comme le benzoate d'alcools en $C_{12}$-$C_{15}$ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Bien entendu, l'homme de l'art veillera à choisir ce ou ces éventuels composés complémentaires et/ou leurs quantités de manière telle que les propriétés avantageuses, en particulier la synergie des facteurs de protection, attachées intrinsèquement aux compositions conformes à l'invention ne soient pas, ou substantiellement pas, altérées par la ou les adjonctions envisagées.

Les compositions de l'invention peuvent être préparées selon les techniques bien connues de l'homme de l'art, en particulier celles destinées à la préparation d'émulsions de type huile-dans-eau ou eau-dans-huile.

Ces compositions peuvent se présenter en particulier sous forme d'émulsion, simple ou complexe (H/E, E/H, H/E/H ou E/H/E) telle qu'une crème, un lait, un gel ou un gel crème, de poudre, de bâtonnet solide et éventuellement être conditionnée en aérosol et se présenter sous forme de mousse ou de spray.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR2315991 et FR2416008).

La composition cosmétique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de poudre, de stick, de mousse aérosol ou de spray.

Lorsque la composition cosmétique selon l'invention est utilisée pour la protection des cheveux contre les rayons UV, elle peut se présenter sous forme de shampooing, de lotion, de gel, d'émulsion, de dispersion vésiculaire non ionique et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95% en poids, de préférence de 70 à 90% en poids, par rapport à l'ensemble de la formulation, la phase huileuse (comprenant notamment les filtres lipophiles) de 5 à 50% en poids, de préférence de 10 à 30% en poids, par rapport à l'ensemble de la formulation, et le ou les (co)émulsionnant(s) de 0,5 à 20% en poids, de préférence de 2 à 10% en poids, par rapport à l'ensemble de la formulation.

Comme indiqué en début de description, un objet de l'invention est l'utilisation d'une composition telle que définie précédemment pour la fabrication d'une composition cosmétique ou dermatologique destinées à la protection de la peau et/ou des cheveux contre le rayonnement ultraviolet, en particulier le rayonnement solaire.

Des exemples concrets, mais nullement limitatifs, illustrant l'invention, vont maintenant être donnés.

| **Exemple 1** | |
|---|---|
| Mélange d'alcool cétylstéarylique et d'alcool cétylstéarylique oxyéthyléné (33 OE) 80/20 (SINNOWAX AO -HENKEL) | 7g |
| Mélange de mono et di-stéarate de glycérol (CERASYNT SD-V ISP) | 2g |
| Alcool cétylique | 1.5g |
| Polydiméthyl siloxane (DOW CORNING 200 FLUID -DOW CORNING) | 1g |
| Benzoate d'alcools en $C_{12}$-$C_{15}$ (WITCONOL TN -WITCO) | 20 g |
| 2-(4-diethylamino-2-hydroxybenzoyl)-benzoate de n-hexyle | 2g |
| Glycérine | 10g |
| Butyl Methoxydibenzoylmethane (PARSOL 1789 HOFFMANN LA ROCHE) | 2g 2g |
| Drometrizole Trisiloxane (SILATRIZOLE, RHODIA CHIMIE) | 2g |
| Conservateurs | qs |
| Eau déminéralisée qsp | 100 g |

| **Exemple 2** | |
|---|---|
| Mélange mono /distéarate de glycerol / stéarate de polyéthylène glycol (100 OE) (ARLACEL 165 FL - ICI) | 2g |
| Alcool stéarylique (LANETTE 18 - HENKEL) | 1g |
| Acide stéarique d'huile de palme (STEARINE TP - STEARINERIE DUBOIS) | 2.5g |
| Poly diméthylsiloxane (DOW CORNING 200 FLUID - DOW CORNING) | 0.5g |
| Benzoate d'alcools en C12/C15 (WITCONOL TN -WITCO) | 18g |
| Triéthanolamine | 0.5g |
| 2-(4-diethylamino-2-hydroxybenzoyl)-benzoate de n-hexyle | 2.5g |
| Drometrisole trisiloxane (SILATRIZOLE - RHODIA CHIMIE) | 2g |
| Butyl methoxydibenzoylmethane (PARSOL 1789 - HOFFMAN - LAROCHE) | 1.5g |
| Glycérine | 5g |
| Phosphate d'alcool hexadécylique,sel de potassium (AMPHISOL K - HOFFMAN LAROCHE) | 1g |
| Acide polyacrylique (SYNTHALEN K - 3V) | 0.3g |
| Hydroxypropyl méthyl cellulose (METHOCEL F4M -DOW CHEMICAL) | 0.1g |
| Triethanolamine | qs pH 7 |
| Conservateurs | qs |
| Eau démineralisée qsp | 100 g |

## Revendications

**1.** Composition cosmétique, en particulier antisolaires, **caractérisée par le fait qu'**elle comprend, dans un support cosmétiquement acceptable :

(i) au moins un dérivé silicié à fonction benzotriazole à titre de premier filtre ;
(ii) au moins un dérivé du dibenzoylméthane à titre de deuxième filtre ; et
(iii) à titre de troisième filtre, au moins un dérivé de 2-hydroxy benzophénone aminosubstitué de formule (III) suivante :

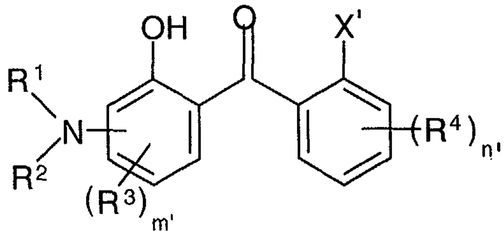

(III)

dans laquelle :

$R^1$ et $R^2$, identiques ou différents, désignent un atome d'hydrogène, un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$ ;
$R^1$ et $R^2$ peuvent également former avec l'atome d'azote avec lequel ils sont liés un cycle à 5 ou 6 chaînons ;
$R^3$ et $R^4$, identiques ou différents, désignent un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$, un radical alcoxy en $C_1$-$C_{12}$, un radical ($C_1$-$C_{20}$)alcoxycarbonyle, un radical alkylamino en $C_1$-$C_{12}$, un radical dialkylamino en $C_1$-$C_{12}$, un radical aryle ou un hétéroaryle éventuellement substitué, un substituant hydrosolubilisant choisi parmi un groupe carboxylate, un groupe sulfonate ou un reste ammonium ;
X' désigne un atome d'hydrogène, un groupe $COOR^5$ ou $CONR^6R^7$;
$R^5$, $R^6$ et $R^7$, identiques ou différents, désignent un atome d'hydrogène, un radical alkyle en $C_1$-$C_{20}$, un radical alcènyle en $C_2$-$C_{10}$, un radical cycloalkyle en $C_3$-$C_{10}$, un radical cycloalcènyle en $C_3$-$C_{10}$, un groupe -(Y'O)o-Z' ou un groupe aryle ;
Y' désigne $-(CH_2)_2-$, $-(CH_2)_3-$ $-(CH_2)_4-$, $-CH-(CH_3)-CH_2-$ ;
Z' représente $-CH_2-CH_3$, $-CH_2CH_2CH_3$, $-CH_2-CH_2-CH_2-CH_3$, $-CH(CH_3)-CH_3$;
m' est un entier variant de 0 à3 ;
n' est un entier variant de 0 à 3 ;
o est un entier variant de 1 à 2.

2. Composition selon la revendication 1, où lesdits premier, second et troisième filtres sont présents dans ladite composition dans une proportion produisant une activité synergique au niveau des facteurs de protection solaire $UVA_{PPD}$ conférés .

3. Composition selon la revendication 1 ou 2, où le dérivé silicié à fonction benzotriazole est choisi parmi les silanes et/ou les polyorganosiloxanes à fonction benzotriazole comprenant au moins une unité de formule (1) suivante :

$$O_{(3-a)/2}\ Si\ (R_7)_a - G \qquad (1)$$

dans laquelle :

- $R_7$ représente un radical alkyle en $C_1$-$C_{10}$ éventuellement halogéné ou un radical phényle ou un radical triméthylsilyloxy,
- a est un nombre entier choisi entre 0 et 3 inclusivement,
- et le symbole G désigne un radical monovalent lié directement à un atome de silicium, et qui répond à la formule (2) suivante :

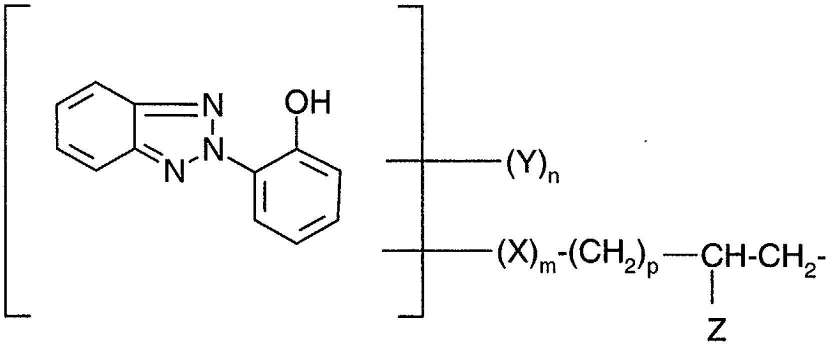 (2)

dans laquelle :

- Y, identiques ou différents, sont choisis parmi les radicaux alkyles en $C_1$-$C_8$, les halogènes et les radicaux alkoxy en $C_1$-$C_4$ étant entendu que, dans ce dernier cas, deux Y adjacents d'un même noyau aromatique peuvent former ensemble un groupement alkylidène dioxy dans lequel le groupe alkylidène contient de 1 à 2 atomes de carbone,
- X représente O ou NH,
- Z représente l'hydrogène ou un radical alkyle en $C_1$-$C_4$,
- n est un nombre entier compris entre 0 et 3 inclusivement,
- m est 0 ou 1,
- p représente un nombre entier compris entre 1 et 10, inclusivement.

**4.** Composition selon la revendication 1 à 3, **caractérisée par le fait que** le dérivé silicié à fonction benzotriazole répond à l'une des formules (5) ou (6) suivantes :

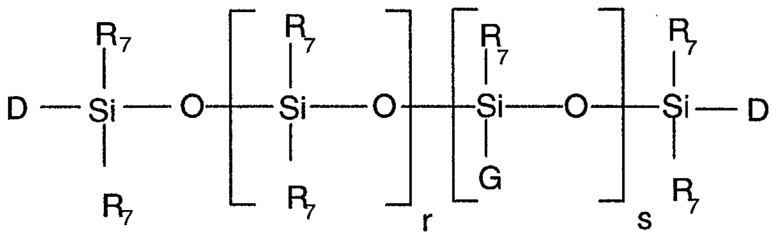 (5)

ou

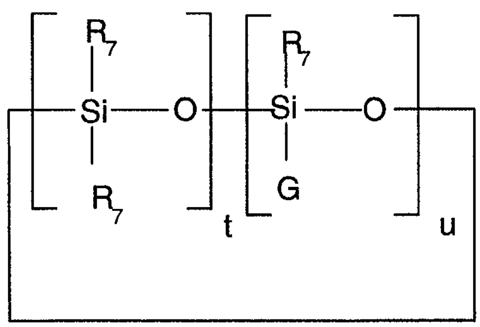 (6)

dans lesquelles :

- $R_7$, identiques ou différents, sont choisis parmi les radicaux alkyles en $C_1$-$C_{10}$, phényle, trifluoro-3,3,3 propyle et triméthylsilyloxy, au moins 80% en nombre des radicaux $R_7$ étant méthyle,
- D, identiques ou différents sont choisis parmi les radicaux $R_7$ et le radical G,
- r est un nombre entier compris entre 0 et 50 inclusivement, et s est un nombre entier compris entre 0 et 20 inclusivement, et si s = 0, au moins l'un des deux symboles D désigne G,
- u est un nombre entier compris entre 1 et 6 inclusivement, et t est un nombre entier compris entre 0 et 10 inclusivement, étant entendu que t + u est égal ou supérieur à 3,
- et le symbole G répond à la formule (2) définie dans la revendication 3.

**5.** Composition selon la revendication 4, **caractérisée par le fait que** le dérivé silicié à fonction benzotriazole répond à la formule (7) suivante :

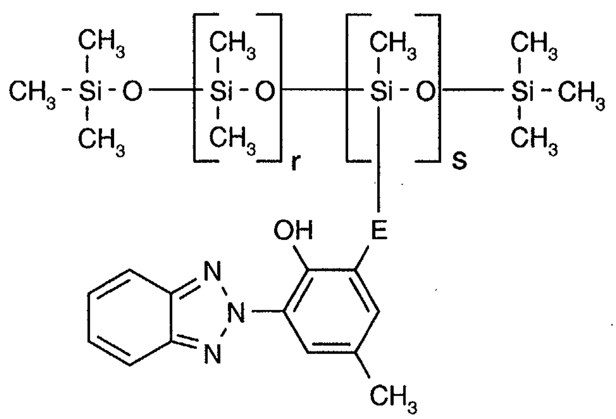

(7)

avec

$$0 \leq r \leq 10,$$

$$1 \leq s \leq 10,$$

et où E représente le radical divalent:

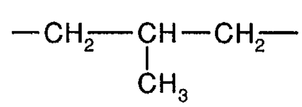

**6.** Composition selon la revendication 5, **caractérisée par le fait que** le dérivé silicié à fonction benzotriazole est le Drométrizole Trisiloxane de formule suivante :

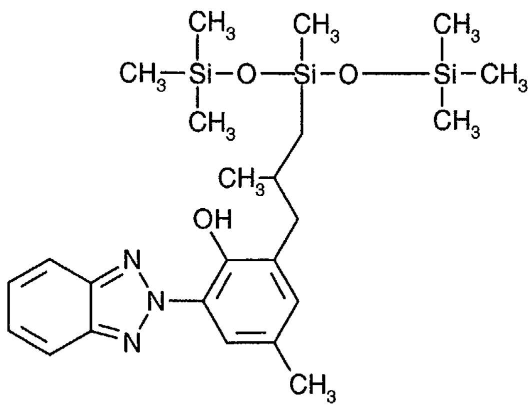

**7.** Composition selon l'une quelconque des revendications 1 à 6, **caractérisée par le fait que** les dérivés siliciés à fonction benzotriazole sont présents à une teneur allant de 0,5% à 20 %, de préférence de 1 % à 10 % en poids, par rapport au poids total de la composition.

**8.** Composition selon l'une quelconque des revendications 1 à 7, **caractérisée par le fait que** le dérivé de dibenzoylméthane est choisi parmi :

- le 2-méthyldibenzoylméthane
- le 4-méthyldibenzoylméthane
- le 4-isopropyldibenzoylméthane
- le 4-tert.-butyldibenzoylméthane
- le 2,4-diméthyldibenzoylméthane
- le 2,5-diméthyldibenzoylméthane
- le 4,4'-diisopropyldibenzoylméthane
- le 4,4'-diméthoxydibenzoylméthane
- le 4-tert.-butyl-4'-méthoxydibenzoylméthane
- le 2-méthyl-5-isopropyl-4'-méthoxydibenzoylméthane
- le 2-méthyl-5-tert.-butyl-4'-méthoxydibenzoylméthane
- le 2,4-diméthyl-4'-méthoxydibenzoylméthane
- le 2,6-diméthyl-4-tert.-butyl-4'-méthoxydibenzoylméthane.

**9.** Composition selon la revendication 8, **caractérisée par le fait que** le dérivé de dibenzoylméthane est le 4-(ter.-butyl) 4'-méthoxy dibenzoylméthane.

**10.** Composition selon la revendication 8, **caractérisée par le fait que** le dérivé de dibenzoylméthane est le 4-isopropyl-dibenzoylméthane.

**11.** Composition selon l'une quelconque des revendications 1 à 10, où le dérivé du dibenzoylméthane est présent à des teneurs allant de 0,5 à 20% en poids et de préférence de 1 % à 10 % en poids, et plus particulièrement de 2 à 8% en poids par rapport au poids total de la composition.

**12.** Composition l'une quelconque des revendications 1 à 11, où le ou les dérivés de 2-hydroxybenzophénone aminosubstitués de formule (III) sont choisis parmi ceux de formule (IIIa) suivante :

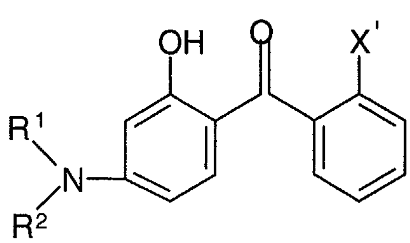

(IIIa)

dans laquelle :

$R^1$ et R , identiques ou différents, désignent un atome d'hydrogène, un radical alkyle en $C_1$-$C_{12}$ ou forment avec l'atome d'azote avec lequel ils sont liés un cycle à 5 ou 6 chaînons ;
X' désigne $COOR^5$ ou $CONR^6R^7$ ;
$R^5$ désigne un atome d'hydrogène, un radical alkyle en $C_1$-$C_{12}$, un radical cycloalkyle en $C_3$-$C_6$.
$R^6$ et $R^7$, identiques ou différents, désignent un atome d'hydrogène, un radical alkyle en $C_1$-$C_{12}$, un radical cycloalkyle en $C_5$-$C_6$.

**13.** Composition selon la revendication 12 où les composés de formule (IIIa) sont sont ceux pour lesquels :

$R^1$ et $R^2$, identiques ou différents, désignent un radical alkyle en $C_1$-$C_4$ et plus particulèrement éthyle ;
$R^5$ désigne un radical alkyle en $C_3$-$C_8$,
$R^6$ et $R^7$, identiques ou différents, désignent un radical alkyle en $C_1$-$C_8$,

**14.** Composition l'une quelconque des revendications 1 à 11, où le ou les dérivés de 2-hydroxybenzophénone aminosubstitués de formule (III) sont choisis parmi ceux de formule (IIIb) suivante :

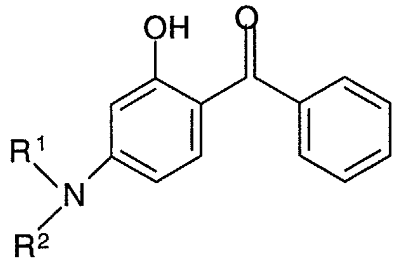

(IIIb)

dans laquelle :

$R^1$ et $R^2$, identiques ou différents, désignent un radical alkyle en $C_1$-$C_{12}$ ou forment avec l'atome d'azote avec lequel ils sont liés un cycle à 5 ou 6 chaînons.

**15.** Composition selon la revendication 14 où les composés de formule (IIIb) sont :

- le (4-diéthylamino-2-hydroxyphényl)-phénylcétone.
- le (4-pyrrolidino-2-hydroxyphényl)-phénylcétone.

**16.** Composition l'une quelconque des revendications 1 à 11, où le ou les dérivés de 2-hydroxybenzophénone aminosubstitués de formule (III) sont choisis parmi ceux de formule (IIIc) suivante :

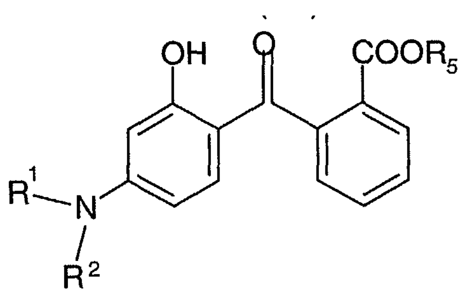

(IIIc)

dans laquelle :

$R^1$ et $R^2$, identiques ou différents, désignent un atome d'hydrogène, un radical alkyle en $C_1$-$C_8$ ou forment avec l'atome d'azote avec lequel ils sont liés un cycle à 5 ou 6 chaînons ;
$R^5$ désigne un atome d'hydrogène, un radical alkyle en $C_1$-$C_{12}$, un radical cycloalkyle en $C_3$-$C_6$.

**17.** Composition selon la revendication 16 où les composés de formule (IIIc) sont choisis parmi :

- le 2-(4-pyrrolidino-2-hydroxybenzoyl)-benzoate
- le 2-(4-diéthylamino-2-hydroxybenzoyl)-benzoate de méthyle
- le 2-(4-diéthylamino-2-hydroxybenzoyl)-benzoate de 2-éthylhexyle
- le 2-(4-diéthylamino-2-hydroxybenzoyl)-benzoate de cyclohexyle
- le 2-(4-diéthylamino-2-hydroxybenzoyl)-benzoate de n-hexyle
- le 2-(4-dibutylamino-2-hydroxybenzoyl)-benzoate
- le 2-(4-dibutylamino-2-hydroxybenzoyl)-benzoate de méthyle
- le 2-(4-dibutylamino-2-hydroxybenzoyl)-benzoate d'isobutyle.

**18.** Composition selon la revendication 17 où le composé de formule (IIIc) est le 2-(4-diéthylamino-2-hydroxybenzoyl)-benzoate de n-hexyle.

**19.** Composition l'une quelconque des revendications 1 à 18, où le ou les dérivés de 2-hydroxybenzophénone aminosubstitués de formule (III) sont présents dans des proportions allant de 0,1 à 20 % en poids par rapport au poids total de la composition.

**20.** Composition selon la revendication 19 où le ou les dérivés de 2-hydroxybenzophénone aminosubstitués de formule (III) sont présents dans des proportions allant de 0,1 à 15% en poids par rapport au poids total de la composition.

**21.** Composition selon la revendication 20, où le ou les dérivés de 2-hydroxybenzophénone aminosubstitués de formule (III) sont présents dans des proportions allant de 0,5 à 10% en poids par rapport au poids total de la composition.

**22.** Composition selon l'une quelconque des revendications 1 à 21, **caractérisée par le fait qu'**elle contient en plus d'autres filtres organiques actifs dans l'UV-A et/ou l'UV-B.

**23.** Composition selon la revendication 22, où le ou les filtres UV organiques complémentaires sont choisis parmi les anthranilates ; les dérivés cinnamiques ; les dérivés salicyliques, les dérivés du camphre ; les dérivés de triazine ; les dérivés de la benzophénone autres que ceux de formule (III) ; les dérivés de β,β-diphénylacrylate, les dérivés de benzotriazole ; les dérivés de benzalmalonate ; les dérivés de benzimidazole ; les imidazolines ; les dérivés bis-benzoazolyle ; les dérivés de l'acide p-aminobenzoïque (PABA) ; les dérivés de méthylène bis-(hydroxyphényl benzotriazole) ; les polymères filtres et silicones filtres autres que les dérivés siliciés de benzotriazole ; les dimères dérivés d'α-alkylstyrène ; les dérivés de 4,4-diarylbutadiène et leurs mélanges.

**24.** Composition selon la revendication 23, **caractérisée par le fait que** le ou les filtres UV organiques sont choisis parmi les composés suivants :

- Ethylhexyl Salicylate,
- Butyl Methoxydibenzoylmethane,
- Ethylhexyl Methoxycinnamate,
- Octocrylene,
- Phenylbenzimidazole Sulfonic Acid,
- Terephthalylidene Dicamphor Sulfonic,.
- Benzophenone-3,
- Benzophenone-4,
- Benzophenone-5,
- 4-Methylbenzylidene camphor,
- Disodium Phenyl Dibenzimidazole Tetra-sulfonate
- Anisotriazine,
- Ethylhexyl triazone,
- Diethylhexyl Butamido Triazone,
- la 2,4,6-tris-(4'-amino benzalmalonate de diisobutyle)-s- triazine.
- Methylene bis-Benzotriazolyl Tetramethylbutylphenol,
- le 1,1-dicarboxy (2,2'-diméthyl-propyl)-4,4-diphénylbutadiène

et leurs mélanges.

**25.** Composition selon l'une quelconque des revendications 1 à 24, **caractérisée par le fait qu'**elle comprend en outre, des pigments ou des nanopigments d'oxydes métalliques, enrobés ou non.

**26.** Composition selon la revendication 25, **caractérisée par le fait que** lesdits pigments ou nanopigments sont choisis parmi les oxydes de titane, de zinc, de fer, de zirconium, de cérium et leurs mélanges, enrobés ou non.

**27.** Composition selon l'une quelconque des revendications 1 à 26, **caractérisée par le fait qu'**elle comprend en outre au moins un agent de bronzage et/ou de brunissage artificiel de la peau.

**28.** Composition selon l'une quelconque des revendications 1 à 27, **caractérisée par le fait qu'**elle comprend en outre au moins un adjuvant choisi parmi les corps gras, les solvants organiques, les épaississants ioniques ou non ioniques, les adoucissants, les antioxydants, les agents anti radicaux libres, les opacifiants, les stabilisants, les émollients, les silicones, les $\alpha$-hydroxyacides, les agents anti-mousse, les agents hydratants, les vitamines, les agents répulsifs contre les insectes, les parfums, les conservateurs, les tensioactifs, les antiinflammatoires, les antagonistes de substance P, les charges, les polymères, les propulseurs, les agents alcalinisants ou acidifiants, les colorants.

**29.** Composition selon l'une quelconque des revendications 1 à 28, **caractérisée par le fait qu'**il s'agit d'une composition protectrice de l'épiderme humain ou d'une composition antisolaire et qu'elle se présente sous forme d'une dispersion vésiculaire non ionique, d'une émulsion, en particulier d'une émulsion de type huile-dans-eau, d'une crème, d'un lait, d'un gel, d'un gel crème, d'une suspension, d'une dispersion, d'une poudre, d'un bâtonnet solide, d'une mousse ou d'un spray.

**30.** Composition selon l'une quelconque des revendications 1 à 29, **caractérisée par le fait qu'**il s'agit d'une composition de maquillage des cils, des sourcils ou de la peau et qu'elle se présente sous forme solide ou pâteuse, anhydre ou aqueuse, d'une émulsion, d'une suspension ou d'une dispersion.

**31.** Composition selon l'une quelconque des revendications 1 à 29, **caractérisée par le fait qu'**il s'agit d'une composition destinée à la protection des cheveux contre les rayons ultraviolets et qu'elle se présente sous la forme d'un shampooing, d'une lotion, d'un gel, d'une émulsion, d'une dispersion vésiculaire non ionique.

**32.** Utilisation d'une composition telle que définie dans les revendications 1 à 29 pour la fabrication de compositions cosmétiques ou dermatologiques destinées à la protection de la peau et/ou des cheveux contre le rayonnement ultraviolet, en particulier le rayonnement solaire.

**33.** Utilisation d'un dérivé de 2-hydroxybenzophénone aminosubstitué tel que défini dans les revendications précédentes pour la fabrication de compositions cosmétiques ou dermatologiques destinées à la protection de la peau et/ou des cheveux contre le rayonnement ultraviolet, en particulier le rayonnement solaire comprenant au moins un filtre du type dérivé silicié de benzotriazole et un filtre du type dérivé de dibenzoylméthane dans le but de produire un effet synergique au niveau des facteurs de protection solaire $UVA_{PPD}$ conférés.

Office européen des brevets

# RAPPORT DE RECHERCHE EUROPEENNE

Numéro de la demande
EP 02 29 2837

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A<br><br><br><br><br>D | EP 1 133 980 A (BASF AG)<br>19 septembre 2001 (2001-09-19)<br>* page 5, ligne 54 - page 9, ligne 51; page 16, ligne 32 - page 19, ligne 8; exemples 6, 7, 11 *<br>& DE 100 12 408 A<br>20 septembre 2001 (2001-09-20)<br>--- | 1-9, 11-13, 16-33 | A61K7/42 |
| A,D | EP 0 660 701 B (L'OREAL)<br>11 juin 1997 (1997-06-11)<br>* le document en entier *<br>--- | 1,3-9, 25-33 | |
| A | WO 97 37634 A (L'OREAL)<br>16 octobre 1997 (1997-10-16)<br>* le document en entier *<br>----- | 1,3-9, 25-33 | |
| | | | DOMAINES TECHNIQUES RECHERCHES (Int.Cl.7) |
| | | | A61K |

Le présent rapport a été établi pour toutes les revendications

| Lieu de la recherche | Date d'achèvement de la recherche | Examinateur |
|---|---|---|
| BERLIN | 19 février 2003 | Van Amsterdam, L |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 02 29 2837

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

19-02-2003

| Document brevet cité au rapport de recherche | Date de publication | Membre(s) de la famille de brevet(s) | Date de publication |
|---|---|---|---|
| EP 1133980 A | 19-09-2001 | DE 10012408 A1 | 20-09-2001 |
| | | AU 2800301 A | 20-09-2001 |
| | | BR 0101085 A | 06-11-2001 |
| | | CN 1324610 A | 05-12-2001 |
| | | EP 1133980 A2 | 19-09-2001 |
| | | JP 2001261540 A | 26-09-2001 |
| | | US 2002001570 A1 | 03-01-2002 |
| EP 660701 B | 05-07-1995 | FR 2695560 A1 | 18-03-1994 |
| | | AU 678382 B2 | 29-05-1997 |
| | | AU 4822493 A | 12-04-1994 |
| | | DE 69311561 D1 | 17-07-1997 |
| | | DE 69311561 T2 | 02-10-1997 |
| | | EP 0660701 A1 | 05-07-1995 |
| | | GR 3024469 T3 | 28-11-1997 |
| | | JP 8504184 T | 07-05-1996 |
| | | US 5618520 A | 08-04-1997 |
| | | AT 154233 T | 15-06-1997 |
| | | CA 2144988 A1 | 31-03-1994 |
| | | DK 660701 T3 | 19-01-1998 |
| | | ES 2102678 T3 | 01-08-1997 |
| | | WO 9406404 A1 | 31-03-1994 |
| WO 9737634 A | 16-10-1997 | FR 2747037 A1 | 10-10-1997 |
| | | AU 692734 B2 | 11-06-1998 |
| | | AU 2393597 A | 29-10-1997 |
| | | BR 9706575 A | 20-07-1999 |
| | | CA 2219863 A1 | 16-10-1997 |
| | | CZ 9800021 A3 | 12-08-1998 |
| | | EP 0835094 A1 | 15-04-1998 |
| | | WO 9737634 A1 | 16-10-1997 |
| | | HU 9900608 A2 | 28-07-1999 |
| | | JP 3359643 B2 | 24-12-2002 |
| | | JP 2001525792 T | 11-12-2001 |
| | | KR 256840 B1 | 15-05-2000 |
| | | NO 975611 A | 05-02-1998 |
| | | NZ 328975 A | 28-01-2000 |
| | | PL 323564 A1 | 14-04-1998 |
| | | US 5955061 A | 21-09-1999 |
| | | ZA 9702594 A | 20-10-1997 |

EPO FORM P0460

Pour tout renseignement concernant cette annexe : voir Journal Officiel de l'Office européen des brevets, No.12/82